(19) Europäisches Patentamt
European Patent Office
Office européen des brevets

(11) Numéro de publication : **0 522 944 A2**

(12) # DEMANDE DE BREVET EUROPEEN

(21) Numéro de dépôt : **92401929.2**

(51) Int. Cl.$^5$ : **A61K 31/44**

(22) Date de dépôt : **06.07.92**

(30) Priorité : **09.07.91 FR 9108601**

(43) Date de publication de la demande : **13.01.93 Bulletin 93/02**

(84) Etats contractants désignés : **PT**

(71) Demandeur : **RHONE-POULENC RORER SA
20, avenue Raymond Aron
F-92160 Antony (FR)**

(72) Inventeur : **Bousseau, Anne
19 rue Daval
F-75011 Paris (FR)**

(74) Mandataire : **Savina, Jacques
Rhône-Poulenc Rorer S.A. Direction Brevets
(t144) 20 avenue Raymond Aron
F-92165 Antony Cédex (FR)**

(54) **Utilisation du (pyridyl-3)-3-1H,3H-pyrrolo 1,2-c thiazole-carboxamide-7 pour le traitement des infections à rétrovirus.**

(57) Application du (pyridyl-3)-3 1H,3H-pyrrolo[1,2-c]thiazole carboxamide-7 de formule :

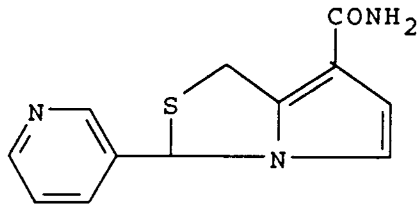

sous ses formes stéréisomères ou leurs mélanges, ainsi que ses sels, pour l'obtention d'un médicament destiné à la prophylaxie et/ou au traitement thérapeutique des infections à rétrovirus.

EP 0 522 944 A2

La présente invention concerne une nouvelle application thérapeutique du (pyridyl-3)-3 1H,3H-pyrrolo[1,2-c] thiazole carboxamide-7 de formule :

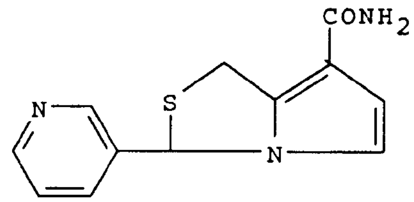

(I)

sous ses formes stéréoisomères ou leur mélange ainsi que ses sels.

Le produit de formule (I) a été précédemment décrit, ainsi que sa préparation, dans les brevets européen EP 115 979 et américain US 4 906 757, pour son utilité dans le traitement des allergies, de l'inflammation et de l'inhibition de l'agrégation des plaquettes sanguines, ainsi que dans le traitement des affections dans lesquelles est impliqué le rôle physiologique du PAF-acéter.

Le dérivé de pyrrolothiazole carboxamide de formule (I) ainsi que ses sels et ses isomères est utile pour l'obtention d'un médicament destiné à la prophylaxie et/ou au traitement thérapeutique des infections à rétrovirus, et plus particulièrement du SIDA (syndrome d'immuno-déficience acquise) et de syndromes associés [ARC (AIDS related complex)].

Par prophylaxie nous sous entendons le traitement des sujets qui ont été exposés aux virus HIVs (human immunodeficiency virus), en particulier les séropositifs asymptomatiques, qui présentent le risque de développer la maladie dans les mois et les années à venir après la primo-infection.

L'activité du produit de formule (I) a été mise en évidence de la manière suivante :

Le Tumour Necrosis Factor (TNF) est responsable de l'activation du virus HIV notamment dans les cellules chroniquement infectées.

Les effets du (+) (pyridyl-3)-3 1H,3H-pyrrolo[1,2-c] thiazolecarboxamide-7 sur l'induction du virus HIV-1 ont été étudiés sur des lignées cellulaires chroniquement infectées.

On utilise des lignées cellulaires U1 obtenues après infection de la lignée promonocytaire, U937, par le virus HIV-1 et sélectionnée le selon la capacité à augmenter la production virale en réponse au Phorbol Myristate Acétate (PMA), au TNF et à d'autres médiateurs [Folks et al., Science, 238, 800 (1987)]. L'activité de la reverse transcriptase est utilisée comme un indicateur de la production virale. On analyse ainsi l'effet de concentrations croissantes du produit à étudier sur des lignées cellulaires stimulées.

Etude expérimentale

Les cultures cellulaires sont prélévées en phase de croissance exponantielle et remises en culture à raison d'une concentration finale de $2x10^5$ cellules/ml, en présence de différentes concentrations du produit à étudier. 30 minutes après, l'ensemble des cultures est additionné de TNF$\alpha$ ou de GM-CSF (Granulocyte Macrophage Colony Stimulating Factor) plus Il-6 (Interleukine-6). Chaque essai est fait en double, excepté les contrôles qui sont faits en quadruple. Chaque jour suivant, une fraction de surnageant des cultures est prélevée, et congelée en vue de la mesure de la reverse transcriptase. Les cellules sont nourries si nécessaire, avec du milieu frais ne contenant ni produit à étudier, ni activateur.

La mesure de l'activité de la reverse transcriptase est faite par les techniques connues, en double exemplaire [Strebel et al., Nature, 328, 728 (1987)].

Le (+) (pyridyl-3)-3 1H,3H-pyrrolo[1,2-c]thiazole carboxamide-7 est étudié aux concentrations de 100, 10, 1 et 0,1 $\mu$M.

Le TNF$\alpha$ est additionné à raison de 100, 10 ou 1 Unités/ml. Le GM-CSF + Il-6 est additionné à raison de 100 U/ml. Certains contrôles ne recoivent pas l'activateur. D'autres contrôles ne reçoivent pas le produit à étudier. D'autres ne reçoivent ni le produit ni l'activateur.

Resultats :

La diminution de la production virale par le (+) (pyridyl-3)-3 1H,3H-pyrrolo[1,2-c]thiazole carboxamide-7 est significative et dose-dépendante dans le cas de cellules U1 traitées par le TNF$\alpha$. Le jour 3, on observe une diminution de 50% de l'activité de la reverse transcriptase pour les cellules U1 traitées par 100 Unités/ml de TNF$\alpha$ et additionnées d'une concentration de $10^{-5}$M du produit ci-dessus et une diminution de 84% dans le cas d'une concentration de $10^{-4}$M.

La diminution de la production virale augmente jusqu'à devenir évidente au jour 7.

L'action inhibitrice du HIV-1 dans les cellules U1, stimulée par le GM-CSF + Il-6 est également significative.

Par ailleurs le (+) (pyridyl-3)-3 1H,3H-pyrrolo[1,2-c] thiazolecarboxamide-7 est sans effet sur la viabilité des cellules quelque soit la concentration utilisée.

Dans une seconde série d'essais, la durée d'action du (+) (pyridyl-3)-3 1H,3H-pyrrolo[1,2-c]thiazolecarboxamide-7 a été testée. Il a ainsi été démontré que des prétraitements pendant des durées allant de 30 minutes à 24 heures avant la stimulation par le TNF, le PMA ou le GM-CSF + Il-6 n'affecte pas l'effet inhibiteur de la réplication du HIV-1.

Les résultats figurent dans les tableaux I, II et III et sont illustrés par les figures 1, 2 et 3.

Tableau I

| Conditions de culture | Production de Reverse Transcriptase Coups par minute (cpm) | | | |
|---|---|---|---|---|
| | Temps de contact avec le produit avant la stimulation (heures) | | | |
| | 0,5 | 2 | 7 | 10 |
| Contrôle (cellules U1) | <100 | <100 | <100 | <100 |
| Cellules U1 + produit (100µM) | <100 | <100 | <100 | <100 |
| Cellules U1 + TNFα(100U) | 1500 | 1000 | 800 | 1200 |
| Cell.U1 + produit (100µM) + TNF α(100U) | 300 | 200 | 180 | env.100 |

Tableau II

| Conditions de culture | Production de Reverse Transcriptase Coups par minute (cpm) — Temps de contact avec le produit avant la stimulation (heures) | | | |
|---|---|---|---|---|
| | 0,5 | 2 | 7 | 10 |
| Contrôle (cellules U1) | env.100 | env.100 | <100 | <100 |
| Cellules U1 + produit (100µM) | env.100 | env.100 | env.100 | <100 |
| Cellules U1 + PMA ($10^{-7}$M) | 6700 | 6000 | 7800 | 6700 |
| Cell.U1 + produit (100µM) + PMA ($10^{-7}$) | 4700 | 1900 | 1900 | 900 |

Tableau III

| Conditions de culture | Production de Reverse Transcriptase Coups par minute (cpm) — Temps de contact avec le produit avant la stimulation (heures) | | | |
|---|---|---|---|---|
| | 0,5 | 2 | 7 | 10 |
| Contrôle (cellules U1) | 160 | 180 | 150 | 160 |
| Cellules U1 + produit (100µM) | 180 | 150 | 180 | 160 |
| Cellules U1 + GM-CSF(100U) + IL6(100U) | 1200 | 650 | 500 | 1000 |
| Cell.U1 + produit (100µM) GM-CSF (100U) + IL6 (100U) | 200 | 180 | 170 | 180 |

La présente invention concerne l'obtention d'une composition pharmaceutique contenant le dérivé de pyrrolothiazole carboxamide de formule (I) sous ses formes stéréoisomères ou leur mélanges, éventuellement sous forme de sel, à l'état pur ou sous forme d'association avec un ou plusieurs diluants ou adjuvants compatibles et pharmaceutiquement acceptables.

Les compositions pharmaceutiques selon l'invention sont capables d'inhiber la réplication des rétrovirus

et donc de réduire la progression vers la maladie ou de diminuer sa gravité chez les sujets infectés.

Notamment dans le cas des infections par le HIV, en inhibant la réplication de ce virus, elles sont capables de réduire la progression vers le SIDA ou de diminuer sa gravité chez les sujets infectés.

Les compositions pharmaceutiques selon l'invention sont susceptibles d'empécher ou de ralentir l'évolution des sujets infectés par rétrovirus vers un stade aggravé de la maladie.

Elles peuvent être utilisées à titre préventif ou curatif. Par "préventif" on entend le fait de prévenir l'évolution chez des sujets présentant une immunodéficience et/ou infectés par rétrovirus.

Bien entendu, la constitution de ces compositions sera adaptée au cas particulier du tractus digestif des immunodéprimés.

Les compositions peuvent être utilisées par voie orale, parentérale ou rectale.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent, également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Comme compositions solides pour l'administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que des diluants, par exemple un lubrifiant tel que le stéarate de magnésium.

Comme compositions liquides pour l'administration orale, on peut utiliser des émulsions pharmaceutiquement acceptables, des solutions, des suspensions, des sirops, des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration rectale sont les suppositoires ou les capsules rectales, qui contiennent outre le principe actif, des excipients tels que le beurre de cacao, des glycérides semi-synthétiques ou des polyéthylèneglycols.

D'une manière générale, le médecin déterminera la posologie qu'il estime la plus appropriée en fonction de l'âge, du poids et des facteurs propres au produit et au sujet à traiter. Généralement chez l'adulte les doses sont comprises entre 25 et 500 mg par jour.

La présente invention concerne également les associations constituées du dérivé de pyrrolothiazole carboxamide de formule (I) sous ses formes stéréoisomères ou leur mélanges, et/ou éventuellement sous forme de sel, et d'un autre principe actif connu pour son activité anti-rétrovirus, éventuellement en présence d'excipients pharmaceutiquement acceptables.

Les agents anti-rétrovirus pouvant être associés sont choisis parmi des agents compatibles et inertes vis à vis du dérivé de pyrrolothiazole carboxamide de formule (I). A titre non limitatif ces agents sont choisis parmi des inhibiteurs de la reverse transcriptase [zidovudine (AZT), didanosine (DDI), didéoxycytidine (DDC), TIBO, névirapine, PMEA, HEPT... ], parmi des inhibiteurs de la protéase [comme par exemple le A 77003], ou parmi des inhibiteurs de la protéine tat [comme par exemple le RO 24-7429].

L' exemple suivant donné à titre non limitatif illustre une composition selon l'invention.

Exemple

- (+) (pyridyl-3)-3 1H,3H-pyrrolo[1,2-c]thiazole carboxamide-7....................................5 mg
- Stéarate de magnésium : 1%.................... 2 mg
- ACDISOL : 1%.................................. 2 mg
- Silice colloïdale : 0,5%...................... 1 mg
- Lactose ...................................... 190 mg

Le (+)(pyridyl)-3)-3 1H,3H-pyrrolo[1,2-c]thiazole carboxamide-7 peut être obtenu de la manière suivante :

L'acide (+) (pyridyl-3)-3 1H,3H-pyrrolo[1,2-c]thiazole carboxy-lique-7 (26,11 kg - 110 moles) est mis en suspension, sous azote dans 116 litres de dichlorométhane, puis porté à reflux. Une solution de chlorure de thionyle (19,506 kg - 159 moles) dans 23 litres de dichlorométhane est additionnée en 2 heures 10 minutes, en maintenant le mélange au reflux jusqu'à 3 heures après la fin de l'addition.

Le mélange est refroidi à 20°C, puis additionné en 1 heure 10 minutes, à 90 litres (1060 moles) d'ammoniaque à 20%, en rinçant le réacteur précédent par 20 litres de dichlorométhane. Le mélange est agité pendant une nuit à environ 20°C, puis additionné de 46 litres d'eau et 40 litres de dichlorométhane et refroidi à 3°C pendant 2 heures. Le précipité est filtré puis lavé à l'eau et essoré. Le filtrat est décanté. Après séparation de la phase aqueuse et extraction par 2 fois 40 litres de dichlorométhane, les phases organiques sont réunies.

Après purification, on obtient 19,6 kg de (+) (pyridyl-3)-3 1H,3H-pyrrolo[1,2-c]thiazole carboxylique-7. L'acide (+) (pyridyl-3)-3 1H,3H-pyrrolo [1,2-c] thiazole carboxylique-7 peut être préparé comme décrit dans le brevet US 4 906 757.

## Revendications

**1 -** Application du (pyridyl-3)-3 1H,3H-pyrrolo[1,2-c]thiazole carboxamide-7 de formule :

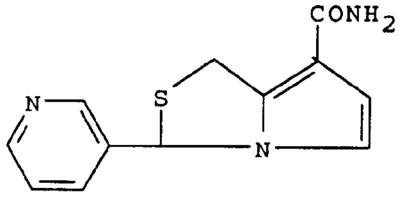

sous ses formes stéréisomères ou leurs mélanges, ainsi que ses sels, pour l'obtention d'un médicament destiné à la prophylaxie et/ou au traitement thérapeutique des infections à rétrovirus.

**2 -** Application selon la revendication 1, caractérisée en ce que le médicament est destiné à la prophylaxie et/ou au traitement du SIDA.

**3 -** Procédé de préparation d'une composition pharmaceutique destinée au traitement préventif et/ou curatif des infections à rétrovirus caractérisé en ce que l'on mélange un produit selon la revendication 1 avec un ou plusieurs diluants ou adjuvants compatibles et pharmaceutiquement acceptables.

**4 -** Association d'un principe actif destiné au traitement du SIDA ou de syndromes analogues constituée du dérivé de pyrrolothiazole carboxamide selon la revendication 1, sous ses formes stéréoisomères ou leur mélanges, et/ou éventuellement sous forme de sel, et d'un autre principe actif connu pour son activité anti-rétrovirus, éventuellement en combinaison avec des excipients inertes et pharmaceutiquement acceptables.

Figure 1

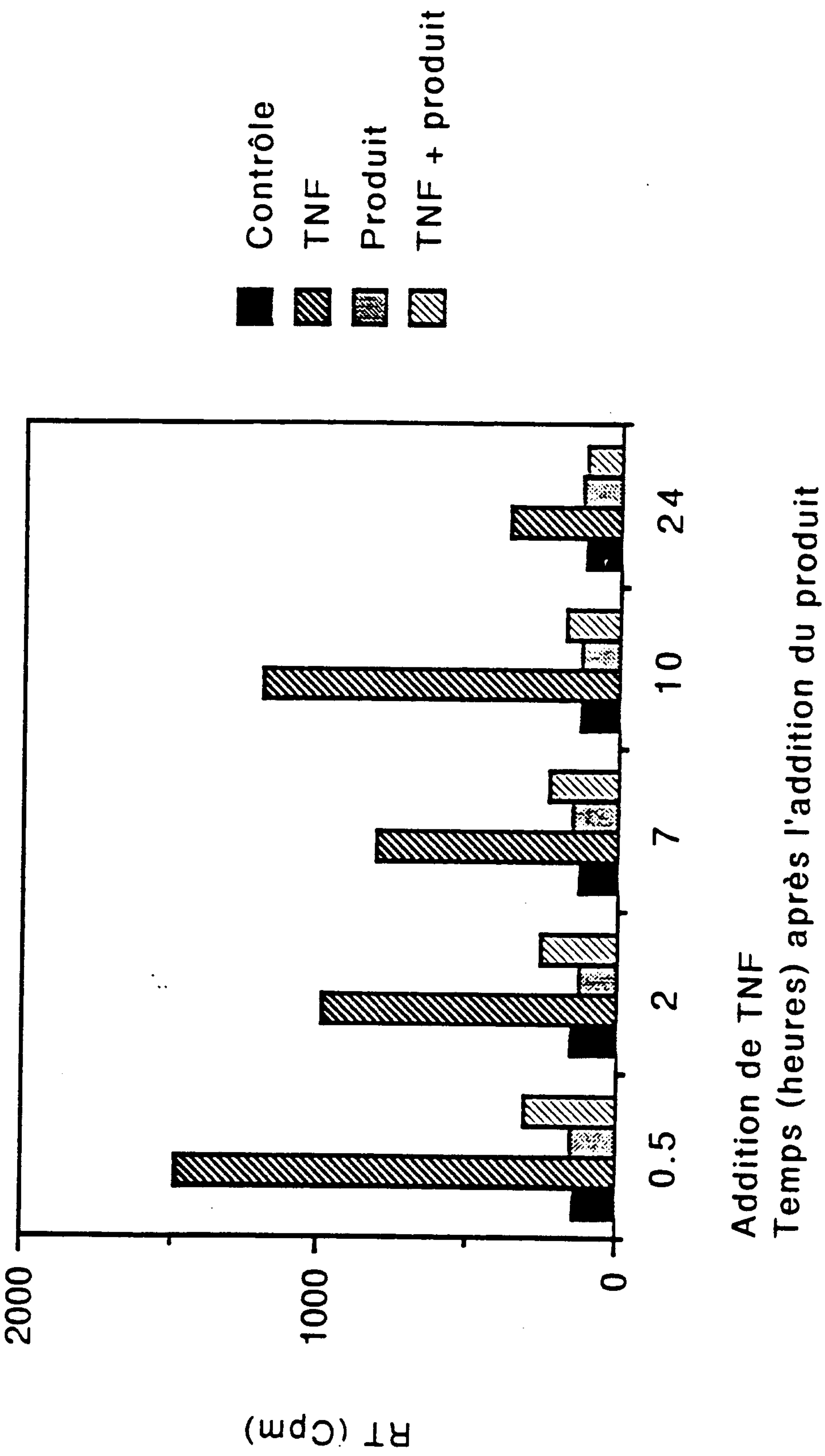
Contrôle
TNF
Produit
TNF + produit
2000
1000
0
RT (Cpm)
0.5
2
7
10
24
Addition de TNF
Temps (heures) après l'addition du produit

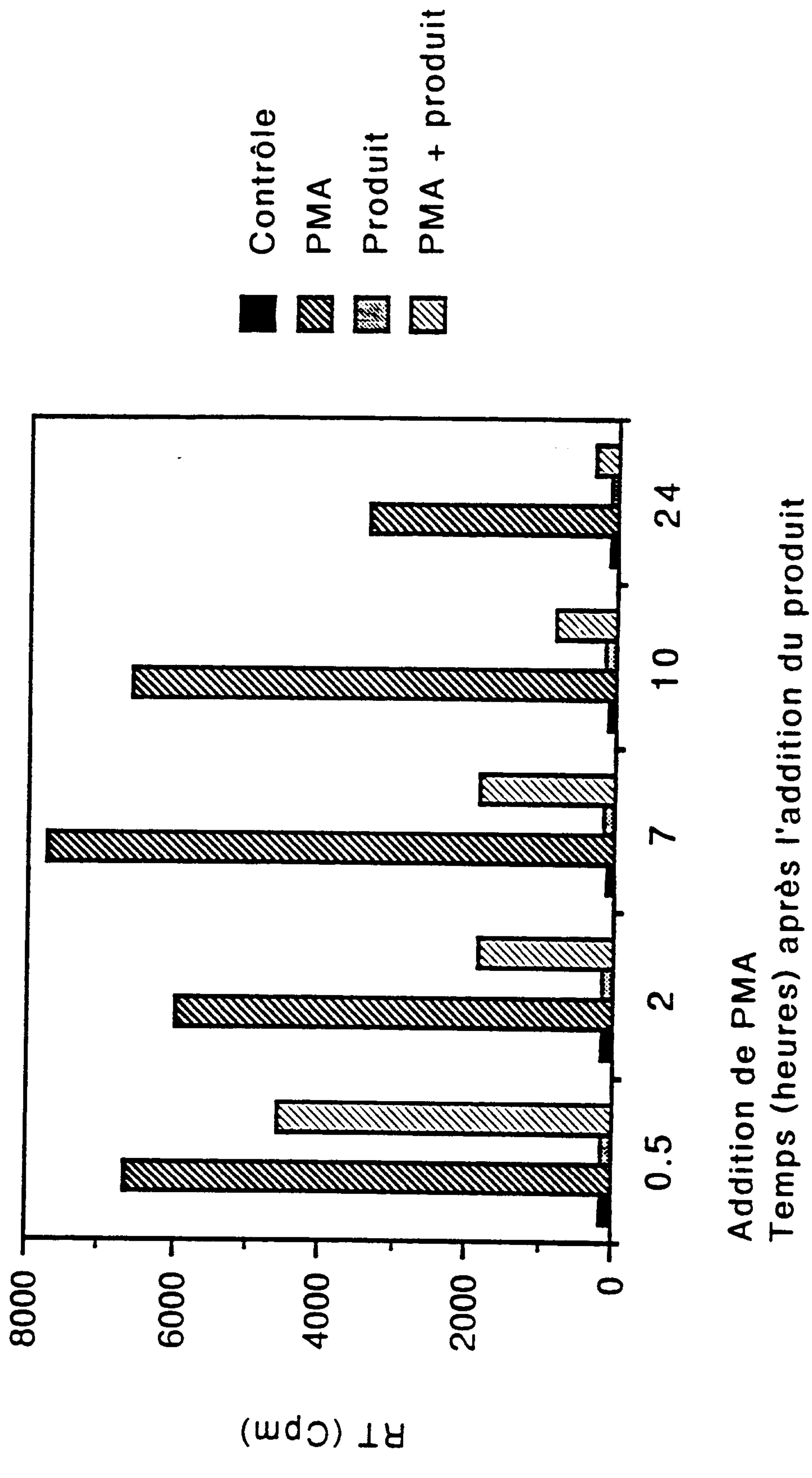
Contrôle
PMA
Produit
PMA + produit
8000
6000
4000
2000
0
RT (Cpm)
0.5
2
7
10
24
Addition de PMA
Temps (heures) après l'addition du produit

Figure 2

Figure 3

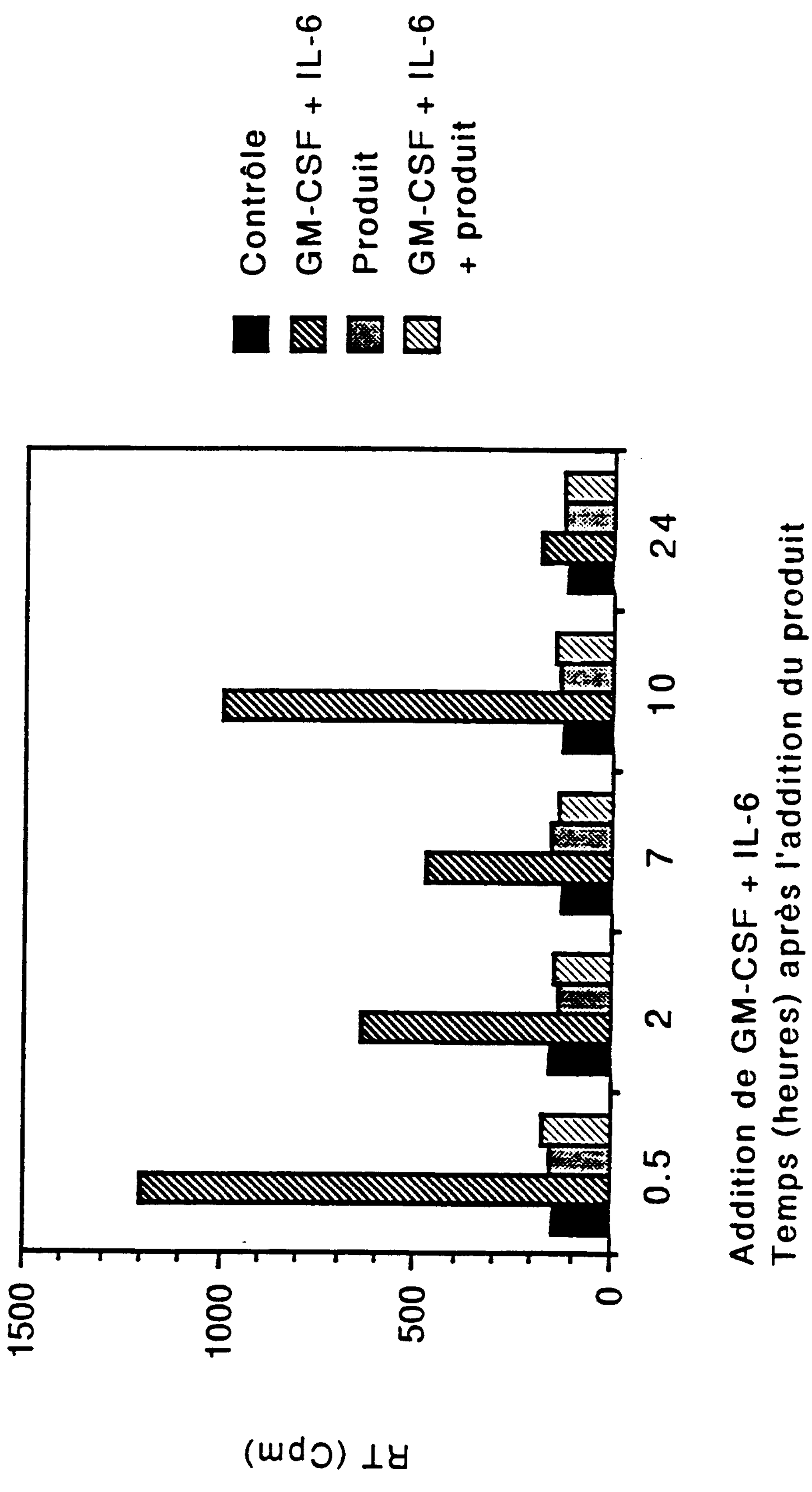

RT (Cpm)
1500
1000
500
0
0.5
2
7
10
24
Addition de GM-CSF + IL-6
Temps (heures) après l'addition du produit
Contrôle
GM-CSF + IL-6
Produit
GM-CSF + IL-6 + produit